# EUROPÄISCHE PATENTSCHRIFT

(11) **EP 0 315 665 B1**
(45) Veröffentlichungstag und Bekanntmachung des Hinweises auf die Patenterteilung: **15.12.1993**
(21) Anmeldenummer: 88904957.3
(22) Anmeldetag: 24.05.1988
(51) Int. Cl.: B01F 13/00

(54) **MISCHVORRICHTUNG**
MIXING APPARATUS
MELANGEUR

(30) Priorität: 21.05.1987 DE 3717134
(43) Veröffentlichungstag der Anmeldung: 17.05.1989
(73) Patentinhaber: Draenert, Klaus, Dr.med.Dr.med.habil., D-81545 München (DE)
(72) Erfinder: Draenert, Klaus, Dr.med.Dr.med.habil., D-81545 München (DE)
(74) Vertreter: VOSSIUS & PARTNER
(86) Internationale Anmeldenummer: EP8800458
(87) Internationale Veröffentlichungsnummer: WO8809209

(56) Entgegenhaltungen:
- WO-A-87/05492
- DE-A- 1 904 343
- DE-A- 2 513 068
- DE-B- 1 057 752
- GB-A- 932 505
- US-A- 1 412 401
- US-A- 4 561 782

## Beschreibung

Die Erfindung betrifft eine Mischvorrichtung zum Rühren und Mischen eines aus mindestens zwei Bestandteilen bestehenden Stoffgemisches, insbesondere zum Mischen von Knochenzement.

Unter Mischen versteht man allgemein das Einbringen von Teilen eines Stoffes zwischen die Teile anderer Stoffe. Ziel ist dabei eine möglichst homogene Verteilung der verschiedenen Bestandteile des Stoffgemisches, um beispielsweise eine chemische Reaktion zwischen den Bestandteilen einzuleiten oder zu fördern, z.B. die nachfolgende Polymerisation eines Zwei- oder Mehrkomponentengemisches.

Es sind verschiedene Arten von Mischverfahren und -vorrichtungen bekannt. Das Mischen erfolgt dabei beispielsweise durch Rühren, Mengen, Walzen, Kneten, Emulgieren, Suspendieren, Lösen oder durch Einwirkung von Ultraschall.

Die bekannten Mischverfahren und -vorrichtungen weisen jedoch den Nachteil auf, daß Verunreinigungen, beispielsweise Luft, in das Mischsystem eingetragen werden können und daß die bereits zu Beginn des Mischvorganges in den zu mischenden Stoffen enthaltenen und die während des Mischvorganges, beispielsweise durch chemische Reaktionen entstehenden Gaseinschlüsse nicht aus dem Mischsystem entfernt werden können.

Spezielle Probleme treten beim Verarbeiten und Mischen von Knochenzement auf.

Als Knochenzement werden meist kalt polymerisierende Zweikomponenten-Kunststoffe verwendet, durch die die Komponenten künstlicher Gelenke im knöchernden Bett verankert werden.

Der Kochenzement härtet unmittelbar nach der Applikation aus und erreicht durch seine plastischen Eigenschaften eine Verblockung der Prothesenkomponente im knöchernen Lager. Seit vielen Jahren werden als Knochenzemente Polymethylmethacrylate (PMMA) verwendet. Diese bestehen aus einem pulverförmigen Perlpolymerisat, welches in flüssigem Monomer angelöst und schließlich durch Polymerisation des Monomers in dieses eingebettet wird. In der Mischphase umfließt das Monomer das etwa kugelförmige Polymerpulver. Dabei gibt es zunächst eine Kugelaufschwemmung, in der mehr oder weniger viele Luftblasen eingeschlossen sind. Die Polymerisation läuft exotherm ab. Neben den eingeschlossenen Luftblasen kommt es regelmäßig beim Umfließen der Polymerkugeln durch das Monomer zu sogenannten "Windschattenphänomenen", also einer unzureichenden Benetzung der Polymerkugeln, und während der exothermen Polymerisation auch zum Verdampfen der monomeren Flüssigkeit, so daß letztlich der ausgehärtete Knochenzement von Blasen unterschiedlicher Ethiologie und Genese durchsetzt ist.

In der Regel wird das Polymerpulver dem Monomer beigegeben und mit einem Spatel in einer Schale vermischt. Es gibt bisher kaum Arbeiten, die sich mit der Misch- oder Quellphase des Knochenzementes befassen.

Die bisher bekannten Versuche, das Problem des Zementmischens in einem sogenannten "geschlossenen System" in den Griff zu bekommen, führten zu keinen besseren Mischungen als die von Hand ausgeführten Ansätze.

Aus der WO 87/5492 (Stand der Technik gemäß Art. 54(3) EPÜ) ist eine Mischvorrichtung bekannt, wie in Fig. 2 dargestellt. Es ist jedoch schwierig, beim Deckel dieser Mischvorrichtung die gewünschte axiale Starrheit bei einem innerhalb der Mischvorrichtung herrschenden Vakuum zu gewährleisten.

Der Erfindung liegt somit die Aufgabe zugrunde, eine evakuierbare Mischvorrichtung bereitzustellen, mit der ein aus mindestens zwei Bestandteilen bestehendes Stoffgemisch einfach, rasch und blasenfrei gerührt und durchmischt werden kann.

Diese Aufgabe wird durch die Merkmale des Patentanspruchs 1 gelöst.

Bei der Anwendung der erfindungsgemäßen Mischvorrichtung zum Mischen von Knochenzement wird zunächst das Monomer in dem Mischbecher der Mischvorrichtung vorgelegt und danach das Polymerpulver in den Ansatz gebracht und unter Vakuum gemischt und verrührt, wie nachstehend noch näher erläutert.

Zum Mischen des Zements wird anstelle herkömmlicher Spatel erfindungsgemäß eine Mischvorrichtung verwendet, die vorzugsweise einen Rundstab aufweist, der vorteilhafterweise mit Teflon beschichtet ist. Der Rundstab hat den Vorteil, daß er das Gemisch nicht zerreißt, sondern dessen Durchmischung durch eine laminare Strömung der Schichten des Gemisches fördert. Außerdem bleibt am Teflonrundstab beim Rühren und Herausziehen praktisch kein Zement haften.

Zur Vermeidung von Lufteinschlüssen wird das Mischen der Bestandteile, beispielsweise der Komponenten des Knochenzementes, unter Vakuum durchgeführt. Hierzu weist der Mischbehälter vorzugsweise einen plangeschliffenen oberen Rand auf, auf den ein Deckel aufgesetzt wird. Die Abdichtung zwischen dem Mischbehälter und dem Deckel kann durch einen Dichtring mit Vakuumfett oder eine Silikonbeschichtung erfolgen. Der Deckel kann auch durch eine rasch lösbare Flanschverbindung mit dem Mischbehälter vakuumdicht verbunden werden. Der Deckel weist einen Anschluß für eine Schlauchzuführung auf, an die eine Vakuumpumpe angeschlossen wird.

Der Deckel für den Mischbecher ist erfindungsgemäß derart ausgebildet, daß er bei Unterdruck, also beim Evakuieren des Mischbechers, im wesentlichen seine Form beibehält und nicht beispielsweise in den Mischbecher hineingezogen wird, daß er aber andererseits so flexibel ist, daß der durch eine Durchführung im Deckel vakuumdicht eingeführte Mischstab in Radialrichtung des Mischbechers bewegt werden kann, so daß durch den Mischstab eine Rührbewegung im evakuierten Mischbecher durchgeführt werden kann. Vorzugsweise ist der Deckel so flexibel, daß der Mischstab parallel an der gesamten Innenwand des Mischbechers entlanggeführt werden kann, zumindest aber an dessen unterem, innerem Rand. In Axialrichtung muß der Deckel hingegen so steif sein, daß er beim Evakuieren nicht zusammengedrückt oder in den Mischbecher hineingezogen wird.

Vorzugsweise weist der Deckel einen festen Rand zur Auflage auf dem Rand des Mischbehälters auf. Die abgedichtete Durchführung für den zum Rühren verwendeten Rundstab ist innerhalb eines in Radialrichtung des Mischbechers flexibel gestalteten Innenteils des Deckels angeordnet, das mit dem festen Rand vakuumdicht, vorzugsweise einstückig, verbunden ist. Aufgrund der Gestaltung des Deckels ist der Rundstab in Radialrichtung im Mischbehälter beweglich und kann an der Innenwand des Mischbehälters entlang geführt werden, so daß kein Teil der zu mischenden Bestandteile, beispielsweise kein Pulver des Knochenzements, unberührt an der Wand liegen bleibt. Dies ist für eine gute Durchmischung außerordentlich wichtig. Andererseits muß der Deckel ausreichend steif sein, daß er bei Unterdruck nicht vollständig zusammengedrückt wird.

Bei einer bevorzugten Ausführungsform ist der Deckel einstückig in Form eines sich nach oben, d.h. vom Mischbecher weg, verjüngenden Balgs ausgebildet. Der Balg besteht vorzugsweise aus mehreren, vorzugsweise drei bis fünf, sich im wesentlichen in Axialrichtung erstreckenden, zylindrischen Abschnitten, deren Durchmesser von Abschnitt zu Abschnitt abnimmt und die jeweils durch ein gekrümmtes Zwischenstück miteinander verbunden sind. Die Zwischenstücke können auch eine etwas dünnere Wandstärke als die axialen Abschnitte aufweisen. Der unterste Abschnitt des Deckels kann dicker und damit steifer als die anderen Abschnitte ausgebildet sein und sitzt als Rand auf dem oberen Rand des Mischbechers auf. Vorzugsweise umgreift der Rand des Deckels den Rand des Mischbechers in Form einer Hinterschneidung derart, daß der Deckel vakuumdicht und im wesentlichen unverschiebbar auf dem Mischbecher aufsitzt. Der oberste Abschnitt des Deckels bildet dabei mit seinem Innendurchmesser die Durchführung für den Mischstab.

Durch die Mischung unter Vakuum können die Blasen im Zement noch weiter reduziert und das Gemisch praktisch blasenfrei gerührt werden.

Bei Versuchen hat sich herausgestellt, daß hohe Mischbecher zu einer sehr viel schnelleren und homogeneren Mischung der Zementmasse führen als weite, flache und an den Ecken totrandige Mischschalen. Vorzugsweise ist der Boden des Mischbechers innen sphärisch oder konkav ausgebildet. Besonders vorteilhaft ist es hierbei, wenn der Innenboden des Mischbechers flexibel ist.

Es kann auch in der Mischphase durch Anwendung von Druck bei gleichzeitiger mechanischer Komprimierung des Zementes ein Vakuum angelegt und Luft aus dem Mischbecher abgesaugt werden, so daß auch kleine Luftblasen bereits während des Mischvorganges zum größten Teil aus dem Zement entfernt werden können.

Vorzugsweise besteht der Mischbecher aus Kunststoff, vorzugsweise einem thermoplastischen Kunststoff, wie einem Polyolefin. Besonders bevorzugt ist die Verwendung von Poly(4-methyl-1-penten) oder TPX®. Es kann auch Polycarbonat verwendet werden.

Der Vakuumdeckel für den Mischbehälter besteht vorzugsweise aus einem sterilisierbaren Material, das weitgehend resistent gegen Lösungsmittel, Mikroorganismen und dergleichen ist.

Wenn der Vakuumdeckel balgförmig ausgebildet ist, besteht er vorzugsweise aus einem Elastomer, wie einem Polyester-Elastomer, Polychloropren-Kautschuk, Polyäthylen, Butylkautschuk (JJR), Nitrilkautschuk (NBR), Butadien-Copolymerisat, Styrol-Butadien-Kautschuk (SBR), Acrylkautschuk, einem Fluorelastomer, einem Polyolefin oder Siliconkautschuk. Besonders bevorzugt ist Hydrel®, ein Polyester-Elastomer, das als Blockpolymer aus Polyterephthalsäureestern und Polyalkenglykolen mit einem Molekulargewicht bis zu 25.000 aufgebaut ist.

Die Erfindung wird nachstehend anhand von Ausführungsbeispielen, die das Mischen und Einfüllen von Knochenzement in einen Applikationsbehälter betreffen, und anhand der Zeichnung näher erläutert. Es zeigen:
- Fig. 1: im Schnitt einen aus der WO 87/5492 bekannten Mischbehälter mit einem zeltförmigen Deckel und einem Rundstab,
- Fig. 2: eine Ausführungsform einer erfindungsgemäßen Mischvorrichtung mit einem als sich verjüngender Balg ausgebildeten und im Schnitt dargestellten Vakuumdeckel, und
- Fig. 3: eine weitere maßstabsgetreue Ausführungsform der Erfindung mit einem balgförmigen Vakuumdeckel im Teilschnitt.

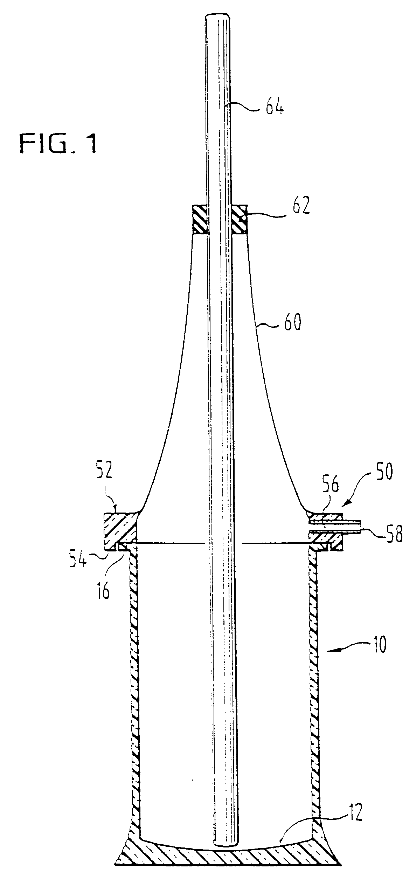

Der Mischbecher 10 gemäß Fig. 1 weist einen konkaven Innenboden 12 und einen plangeschliffenen oberen Rand 16 auf. Zum Durchführen des Rührens und Mischens unter Vakuum weist der Mischbecher 10 gemaß Fig. 1 einen Deckel 50 auf, dessen fester Rand 52 auf dem plangeschliffenen Rand 16 des Mischbechers 10 aufliegt. Zur Abdichtung zwischen den beiden in Kontakt stehenden Flächen der Ränder 16 und 52 kann eine Silikonschicht verwendet werden, es kann aber auch ein Dichtring in einer Nut des Randes 16 vorgesehen sein. Der Rand 52 des Deckels 50 weist außen eine Nase 54 auf, die den Rand 16 übergreift und ein Verschieben des Deckels 50 gegenüber dem Rand 16 des Mischbechers 10 verhindert.

Ferner ist im Rand 52 des Deckels 50 eine Durchführung 56 und ein Stutzen 58 zum Anschluß einer (nicht dargestellten) Vakuumleitung vorgesehen, die zu einer (ebenfalls nicht dargestellten) Pumpe führt.

Neben dem festen Rand 52 weist der Deckel 50 ein flexibles, zeltförmiges Innenteil 60 aus. Das Innenteil 60 ist einstückig mit dem Rand 52 verbunden und besteht aus demselben Material wie der Rand 52, ist allerdings als dessen Fortsetzung dünner ausgezogen, so daß das Material des Innenteils 60 eine gewisse Flexibilität aufweist. Als Material hierfür eignet sich insbesondere Teflon oder Poly(4-methyl-1-penten). Das flexible Innenteil 60 kann aber auch durch eine vakuumdichte Verbindung, beispielsweise eine Flanschverbindung, mit dem festen Rand 52 verbunden sein und aus einem anderen Material als der feste Rand 52 bestehen, beispielsweise aus einer stabilen Kunststoffolie.

Am oberen Ende des dach- oder zeltförmigen Innenteils 60 ist eine ringförmige Durchführung 62 aus einem dehnbaren Material, vorzugsweise einem sterilen Gummi oder Silikon, vakuumdicht eingeschweißt. Das Innenteil 60 mit der Durchführung 62 kann vorgespannt sein, so daß es eine gewisse Stabilität aufweist. Durch die flexible Durchführung 62 ist ein Rundstab 64 geführt, der vorzugsweise aus Teflon besteht und etwa 8 mm dick ist.

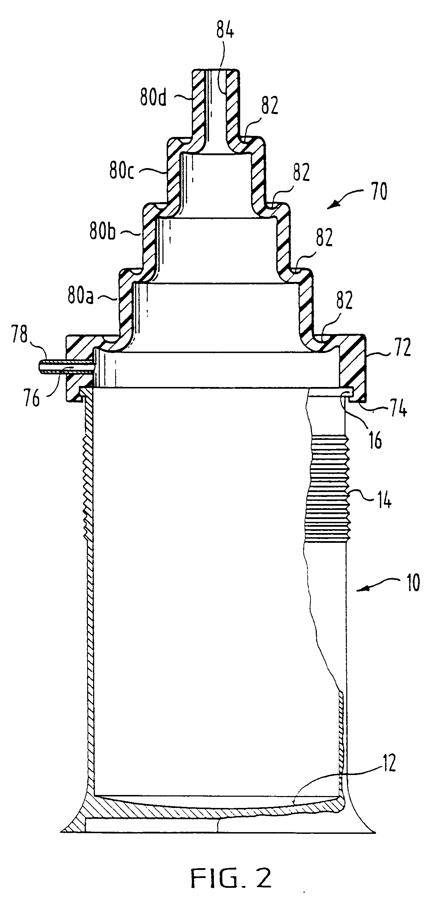

In Fig. 2 ist eine Ausführungsform eines erfindungsgemäßen Vakuumdeckels 70 für den Mischbecher 10 im Querschnitt dargestellt, wobei der Mischbecher 10 teilweise als Ansicht dargestellt ist.

Der Mischbecher 10 gemäß Fig. 2 entspricht dem Mischbecher 10 gemäß Fig. 1, wobei in Fig. 2 noch eine geriffelte Grifffläche 14 vorgesehen ist.

Der Vakuumdeckel 70 ist einstückig ausgebildet und seine Form ist mit der eines sich verjüngenden Balgs vergleichbar. Der Deckel 70 besteht aus mehreren miteinander verbundenen Abschnitten, deren Durchmesser sich allmählich verkleinert. Der unterste Abschnitt 72 hat eine größere Wandstärke als die anderen Abschnitte und dient als Rand, der vakuumdicht auf dem Rand 16 des Mischbechers aufsitzt. Zu diesem Zweck weist der Abschnitt 72 eine Nase 74 auf, die den Rand 16 des Mischbechers in Form einer Hinterschneidung fest umgreift.

Aufgrund der Elastizität des Materials läßt sich der Rand 72 zum Aufsetzen auf den Mischbecher 10 leicht nach außen drücken.

Ferner ist im untersten Abschnitt 72 eine Durchführung 76 für einen (schematisch dargestellten) Stutzen 78 zum Anschluß einer zu einer Pumpe führenden Vakuumleitung vorgesehen.

Der Vakuumdeckel 70 gemäß Fig. 2 weist neben dem untersten Abschnitt 72 vier weitere Abschnitte 80a - d auf, deren Durchmesser allmählich abnimmt und die durch etwas verdünnte bogenförmige Zwischenstücke 82 einstückig miteinander verbunden sind. Aufgrund dieser Ausbildung ist der Vakuumdeckel 70 zwar in Axialrichtung relativ starr und auch unter Vakuum kaum zusammendrückbar, in Radialrichtung kann aber der Deckel relativ leicht bewegt werden, insbesondere dessen oberster Abschnitt 80d, dessen innerer Umfang eine Durchführung 84 für den (in Fig. 2 nicht dargestellten) Mischstab darstellt. Der Innendurchmesser der Durchführung 84 ist vorzugsweise etwas kleiner als der Außendurchmesser des Mischstabs, so daß beim Einführen des Mischstabs die Durchführung 84 etwas ausgeweitet wird und den eingeführten Mischstab weitgehend vakuumdicht festhält.

In Fig. 2 sind die einzelnen Abschnitte 80a - d im wesentlichen kreiszylindrisch und weisen die gleiche Wandstärke auf. Die einzelnen Abschnitte 80 können aber auch nach oben konisch zulaufen, und ihre Wandstärke kann sich gegebenenfalls vom untersten Abschnitt 80a bis zum obersten Abschnitt 80d kontinuierlich verjüngen. Die Zwischenstücke 82 können gegebenenfalls auch die gleiche Wandstärke wie die Abschnitte 80 aufweisen, wenn aufgrund der Wahl des Materials eine ausreichende seitliche Elastizität des gesamten Deckels 70 gewährleistet ist.

Der Deckel 70 sollte insgesamt so elastisch sein, daß der oberste Abschnitt 80d so weit radial beweglich ist, daß der durch die Durchführung 84 geführte Mischstab parallel an der Innenwand des Mischbechers 10 anliegen und an dieser Wand entlanggeführt werden kann.

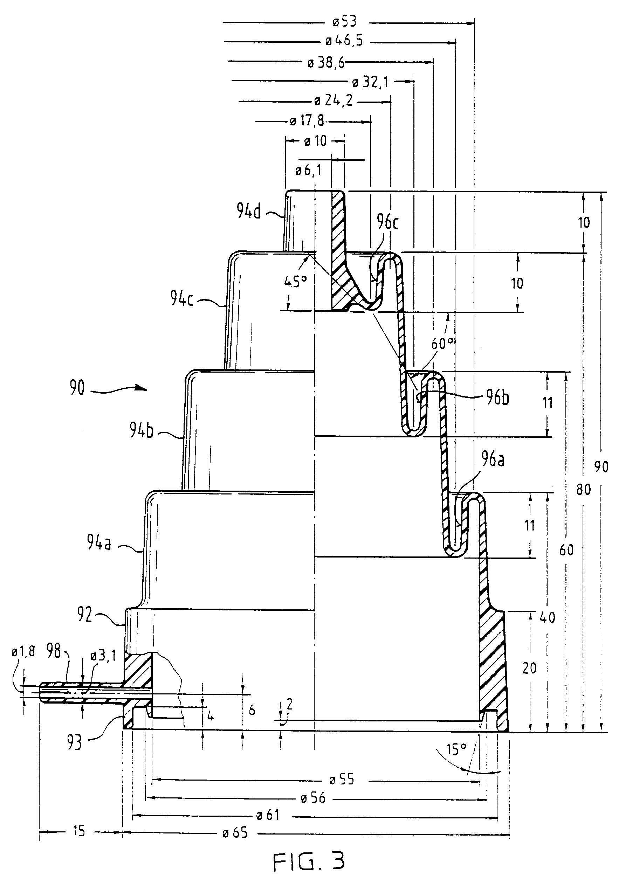

Die federartigen Zwischenstücke 82 können wie in Fig. 3 U-förmig gebogen sein, sie können ggf. auch gerade sein und rechtwinklig zu den einzelnen Abschnitten 80.

Fig. 3 zeigt weitgehend maßstabsgetreu und teilweise geschnitten eine weitere Ausführungsform des balgförmigen Vakuumdeckels. Die Zahlenangaben in Fig. 3 bedeuten "mm", soweit nichts anderes angegeben ist.

Der Vakuumdeckel 90 gemäß Fig. 3 weist einen stabilen untersten Abschnitt 92 auf, der gleichzeitig als Rand dient und mit seinem Vorsprung 93 den oberen Rand des Mischbechers umfaßt und den Vakuumdeckel 90 mit dem (in Fig. 3 nur angedeuteten) Mischbecher vakuumdicht verbindet.

Der unterste Abschnitt 92 verjüngt sich nach oben zu dem nächsten Abschnitt 94a, der wie der Abschnitt 92 im wesentlichen in Axialrichtung verläuft. Die weiteren Abschnitte 94b und 94c weisen im wesentlichen die gleiche Wandstärke auf wie der Abschnitt 94a und verlaufen leicht konisch nach innen geneigt. Zwischen den einzelnen Abschnitten des Vakuumdeckels 90 sind schleifenförmige Zwischenstücke 96a, 96b und 96c vorgesehen, die in Form eines doppelten U ausgebildet sind und ebenfalls die gleiche Wandstärke wie die mittleren Abschnitte des Vakuumdeckels 90 aufweisen. Das oberste Zwischenstück 96c verdickt sich an seinem Ende und geht in den ebenfalls verdickten obersten Abschnitt 94d des Vakuumdeckels 90 über, dessen innerer Umfang die Halterung oder Durchführung für einen (in Fig. 3 nicht dargestellten) runden Mischstab ausbildet. Die Oberfläche des sich verdickenden Teils des Zwischenstücks 96c ist zur Axialrichtung in einem Winkel von etwa 30° und dessen untere Fläche in einem Winkel von etwa 45° geneigt. Dies ist besonders vorteilhaft für die Flexibilität der Durchführung des Mischstabes. Insgesamt ist der Vakuumdeckel 90 mit seinem mäanderförmigen Querschnitt außerordentlich flexibel, so daß der Mischstab auch unter Vakuum leicht zum Rühren des Knochenzements bewegt werden kann. Für die Flexibilität des Vakuumdeckels 90 ist auch die langgestreckte schleifenförmige Querschnittsform in Verbindung mit den Materialeigenschaften besonders vorteilhaft. Als Material für den Vakuumdeckel 90 wird vorzugsweise Hytrel® verwendet.

Am untersten Abschnitt 92 des Vakuumdeckels 90 ist einstückig ein Anschlußstutzen 98 für eine Vakuumleitung vorgesehen.

Die erfindungsgemäße Vorrichtung gemäß Figuren 2 und 3 wird zum Rühren oder Vormischen des Rohgemisches aus Monomer und Polymerpulver im Mischbecher 10 unter Vakuum etwa in den ersten 30 Sekunden der Mischphase verwendet. Ein Rundstab wird verwendet, um ein Haften des Zements beim Rühren und späteren Herausziehen des Stabes zu verhindern und das Gemisch nicht zu zerreißen, sondern eine laminare Strömung der einzelnen Schichten des Gemisches zu erzeugen, durch die die Durchmischung gefördert wird. Nach dem Abschluß der Rühr- und Mischphase wird der Rundstab herausgezogen und der Deckel 70, 90 vom Mischbecher 10 gelöst.

Die Erfindung ist nicht auf die vorstehenden Ausführungsbeispiele beschränkt, die das Mischen von Knochenzement betreffen, sondern kann allgemein zum Mischen eines aus mindestens zwei Bestandteilen bestehenden Stoffgemisches verwendet werden. Die vorstehend beschriebenen Ausführungsbeispiele der erfindungsgemäßen Vorrichtung sind daher allgemein zum Rühren und Mischen von Stoffen anwendbar. Die Weiterverarbeitung, insbesondere das Einfüllen des Stoffgemisches in einen Behälter, kann in der in WO87/5492 beschriebenen Weise erfolgen.

## Patentansprüche

1. Mischvorrichtung mit einem Mischbecher (10) und einem Deckel (70; 90) zum vakuumdichten Verschließen des Mischbechers (10) mit einer Durchführung (84) für einen Mischstab (64) und einer Einrichtung zum Evakuieren des Inneren der Mischvorrichtung, wobei zumindest der die Durchführung (84) für den Mischstab (64) umschließende Teil des Deckels (70; 90) in Radialrichtung des Mischbechers so flexibel ist, daß durch den Mischstab (64) eine Rührbewegung im Inneren der evakuierten Mischvorrichtung durchführbar ist, und der Deckel (70; 90) derart ausgebildet ist, daß er in Axialrichtung des Mischbechers so starr ist, daß er beim Evakuieren des Inneren der Mischvorrichtung im wesentlichen seine Form beibehält.

2. Vorrichtung nach Anspruch 1, dadurch gekennzeichnet, daß der Deckel (70; 90) in Axialrichtung mehrere Abschnitte (80; 94) aufweist, wobei der Durchmesser jedes Abschnitts kleiner ist als der des darunterliegenden Abschnitts und die einzelnen Abschnitte (80; 94) durch flexible Zwischenstücke (82; 96) miteinander verbunden sind.

3. Vorrichtung nach Anspruch 2, dadurch gekennzeichnet, daß die Abschnitte (80; 94) und die Zwischenstücke (82; 96) miteinander einstückig sind.

4. Vorrichtung nach einem der Ansprüche 1 bis 3, dadurch gekennzeichnet, daß der Deckel (70; 90) als sich nach oben verjüngender Balg ausgebildet ist.

5. Vorrichtung nach einem der Ansprüche 1 bis 4, dadurch gekennzeichnet, daß der Deckel (70; 90) aus einem sterilisierbaren Material besteht, vorzugsweise aus einem Polyester-Elastomer.

6. Vorrichtung nach einem der Ansprüche 1 bis 5, dadurch gekennzeichnet, daß der Deckel (70; 90) einen Stutzen (78; 98) zum Anschließen einer Vakuumleitung aufweist.

7. Vorrichtung nach einem der Ansprüche 1 bis 6, dadurch gekennzeichnet, daß der Mischstab (64) ein Rundstab ist.

8. Vorrichtung nach einem der Ansprüche 1 bis 7, dadurch gekennzeichnet, daß der Mischbecher (10) zylindrisch ist und sein Boden (12) innen konkav oder rund ausgebildet ist.

9. Verwendung der Vorrichtung nach einem der Ansprüche 1 bis 8 zum Mischen, insbesondere zum Mischen von Knochenzement unter Vakuum.

## Claims

1. A mixing apparatus with a mixing bowl (10) and a lid (70; 90) for sealing the mixing bowl (10) in a vacuum-tight manner, said lid comprising a feed-through (84) for a mixing rod (64) and a means for evacuating the interior of the mixing apparatus, wherein at least the part of the lid (70; 90) surrounding the feed-through (84) for the mixing rod (64) is flexible in the radial direction of the mixing bowl such that a stirring movement can be effected by means of the mixing rod (64) in the interior of the evacuated mixing apparatus, and wherein the lid (70; 90) is designed such that it is sufficiently rigid in the axial direction of the mixing bowl to essentially retain its shape when the interior of the mixing apparatus is evacuated.

2. The apparatus according to claim 1, characterized in that the lid (70; 90) comprises several sections (80; 94) in axial direction, wherein the diameter of each section is smaller than that of the section below and the individual sections (80; 94) are connected to one another by means of flexible intermediate pieces (82; 96).

3. The apparatus according to claim 2, characterized in that the sections (80; 94) and the intermediate pieces (82; 96) are integral.

4. The apparatus according to any one of claims 1 to 3, characterized in that the lid (70; 90) is designed as an upward tapered bellows.

5. The apparatus according to any one of claims 1 to 4, characterized in that the lid (70; 90) is made of a sterilizable material, preferably of a polyester elastomer.

6. The apparatus according to any one of claims 1 to 5, characterized in that the lid (70; 90) comprises a connecting piece (78; 98) for connection with a vacuum line.

7. The apparatus according to any one of claims 1 to 6, characterized in that the mixing rod (64) is a round rod.

8. The apparatus according to any one of claims 1 to 7, characterized in that the mixing bowl (10) is cylindrical and the interior of the bottom thereof (12) is concave or round.

9. Use of the apparatus according to any one of claims 1 to 8 for mixing, in particular for mixing bone cement under vacuum.

## Revendications

1. Dispositif de mélange comprenant un godet mélangeur (10) et un couvercle (70, 90), destiné à obturer d'une manière étanche au vide le godet mélangeur (10) et présentant un passage (84) pour une barre de mélange (64), ainsi qu'un dispositif pour amener sous vide l'intérieur du dispositif de mélange, au moins la partie du couvercle (70, 90) qui entoure le passage (84) prévu pour la barre de mélange (64) étant flexible dans la direction radiale du godet mélangeur de façon qu'un mouvement d'agitation puisse être effectué, à l'intérieur du dispositif de mélange mis sous vide, par la barre de mélange (64), le couvercle (70, 90) étant réalisé de façon qu'il soit suffisamment rigide dans la direction axiale du godet mélangeur pour qu'il conserve sensiblement sa forme lors de la mise sous vide de l'intérieur du dispositif de mélange.

2. Dispositif suivant la revendication 1, caractérisé en ce que le couvercle (70, 90) présente en direction axiale plusieurs tronçons (80, 94), le diamètre de chaque tronçon étant plus petit que celui du tronçon sous-jacent et en ce que les différents tronçons (80, 94) sont mutuellement reliés par des pièces intermédiaires flexibles (82, 96).

3. Dispositif suivant la revendication 2, caractérisé en ce que les tronçons (80, 94) et les pièces intermédiaires (82, 96) sont ensemble d'une pièce.

4. Dispositif suivant l'une des revendications 1 à 3, caractérisé en ce que le couvercle (70, 90) est réalisé sous la forme d'un soufflet qui s'amincit vers le haut.

5. Dispositif suivant l'une des revendications 1 à 4, caractérisé en ce que le couvercle (70, 90) est constitué d'une matière stérilisable, de préférence d'un élastomère de polyester.

6. Dispositif suivant l'une des revendications 1 à 5, caractérisé en ce que le couvercle (70, 90) présente une tubulure (78, 98) pour le raccordement d'un conduit à vide.

7. Dispositif suivant l'une des revendications 1 à 6, caractérisé en ce que la barre de mélange (64) est une barre à section ronde.

8. Dispositif suivant l'une des revendications 1 à 7, caractérisé en ce que le godet mélangeur (10) est cylindrique et en ce que son fond (12) est réalisé à l'intérieur sous une forme concave ou ronde.

9. Utilisation du dispositif suivant l'une des revendications 1 à 8 pour le mélange, en particulier pour le mélange de ciment pour os sous vide.
